# EUROPEAN PATENT SPECIFICATION

(11) **EP 1 455 689 B1**
(45) Date of publication and mention of the grant of the patent: **21.02.2007**
(21) Application number: 02779803.2
(22) Date of filing: 29.10.2002
(51) Int. Cl.: A61F 2/14

(54) **ORBITAL IMPLANT**
ORBITALIMPLANTAT
IMPLANT ORBITAIRE

(30) Priority: 30.10.2001 ZA 200108961
(43) Date of publication of application: 15.09.2004
(73) Proprietor: Eyeborn (Proprietary) Limited, Pretoria 0184 (ZA)
(72) Inventor: RICHTER, Paul, Wilhelm, 0153 Wingate Park (ZA); THOMAS, Michael Edward, 0081 Pretoria (ZA); TALMA, Jan, 0181 Pretoria (ZA); GOUS, Petrus Nicolaas Jacobus, 0181 Pretoria (ZA); ROUX, Paul, Brooklyn, 0181 Pretoria (ZA); MINNAAR, Mark, Muckleneuk, 0002 Pretoria (ZA); LEVITZ, Lewis Mark, 2090 Johannesburg (ZA)
(74) Representative: Senior, Alan Murray
(86) International application number: PCT/IB2002/004481
(87) International publication number: WO 2003/037155

(56) References cited:
- US-A- 5 089 021
- US-A- 5 330 529
- US-A- 5 466 259
- US-A- 5 584 880
- US-A- 5 713 955

## Description

THIS INVENTION relates to an orbital implant.

According to the invention, there is provided an orbital implant which includes a body of bioactive material having macropores of at least 400*µ*m, and a cap of bioactive material having substantially no pores or only micropores smaller than 50*µ*m, with the cap covering a portion of the body.

The term 'bioactive material' used in this specification has its usual generally accepted meaning or definition, namely that it is 'a material that elicits a specific biological response at the interface of the material which results in the formation of a bond between the tissues and the material', as provided by L.L. Hench and J. Wilson in "An Introduction to-Bioceramics", Advanced Series in Ceramics - Vol. 1, Ed. L.L. Hench and J. Wilson, World Scientific, Singapore, New Jersey, London, Hong Kong (1993) p. 7.

The bioactive material may be a calcium phosphate material or compound such as a hydroxyapatite; a bioactive glass, which can typically be based on SiO₂, NazO, CaO and/or P₂O₅; a bioactive glass ceramic, which can be similar in composition to bioactive glass but which incorporates additionally MgO, CaF₂ and/or metal oxides; or a composite material comprising a polymer containing bioactive material particles, such as particles of a calcium phosphate compound, a bioactive glass and/or a bioactive glass ceramic.

The orbital implant may preferably be spherical. It will thus be of a size so that it can be inserted into, and fit into, the orbit of a mammal, either to replace the contents of an eye following evisceration or to replace the eyeball following enucleation. Thus, when it is to be implanted into the orbit of an adult human, it may have a diameter of about 20mm.

The macropores of the body may be substantially spherical so that they have diameters of said at least 400*µ*m. Preferably, the diameters of the macropores do not exceed 1000*µ*m. An orbital implant having pores of a size from 200 - 600 µm is known from US-A- 5 584 880.

Some macropores may be in communication with the outer surface of the body. In other words, when such macropores are present, the body will have irregularly spaced surface indentations or dimples. Adjacent macropores in the body may be interconnected by openings and/or passageways. Thus, by means of the macropores which are in communication with the body outer surface and the openings and/or passageways between adjacent macropores, open paths to the body outer surface, defined by the macropores, are provided in the implant body. The interconnecting openings or passageways between adjacent macropores may have diameters greater than 50*µ*m, preferably greater than 100*µ*m.

In other words, the body may contain substantially no isolated or closed macropores.

The macropores in the body may occupy-from 40% to 85% by volume, preferably about 60% by volume, of the body.

The body may also have micropores smaller than 50*µ*m. At least so me of these micropores may be of irregular shape. Thus, they may be in the form of interstitial spaces, for example, interstitial spaces between particles of bioactive material, resulting from incomplete sintering of the particles during formation of the body. The sizes of these micropores are then dependent on the sizes of the bioactive material particles from which the body is sintered. However, these micropores will have a maximum dimension smaller than 50*µ*m, and their maximum dimension may typically be of the order of 1*µ*m, or even smaller. Instead, or additionally, at least some of the micropores may be of regular shape, eg substantially spherical so that their diameters are thus smaller than 50µm. The micropores, when present, may occupy from 3% to 70% by volume, preferably about 40% by volume, of the macropore-free bioactive material, ie the residual bioactive material around the macropores.

All the spherical micropores present in the body may be of substantially the same size, while all the irregularly shaped micropores may be of substantially the same size. The irregularly shaped micropores may be smaller than the spherical micropores. For example, when the irregularly shaped micropores are of the order of 1*µ*m or smaller, the spherical micropores may have diameters of at least 10*µ*m, and may typically have diameters of 10-45*µ*m.

Adjacent micropores in the body are then preferably interconnected by openings and/or passageways. Some micropores may also be interconnected to the macropores by openings and/or passageways. The micropores will thus, by means of these openings and/or passageways, provide open paths to the macropores, as well as, together with the macropores, open paths to the outer surface of the body. In other words, there may thus be substantially no isolated or closed micropores in the body.

More specifically, both interstitial micropores and spherical micropores may be present in the body, with adjacent spherical micropores being interconnected by interstitial micropores which thus constitute the interconnecting openings or passageways. The interstitial micropores will then also interconnect the spherical micropores to the macropores.

The body may thus have a trimodal pore size distribution, comprising macropores, which may be in the size range 400-1000µm; larger micropores which may be in the size range smaller than 50*µ*m but at least 10*µ*m; and smaller micropores which are 1*µ*m or smaller.

The cap may, in one embodiment of the invention, be of bioactive material containing substantially no pores. However, in another embodiment of the invention, the cap may contain pores; however, the pores will then be micropores smaller than 50*µ*m, ie the pores will then be irregular micropores and/or spherical micropores, as hereinbefore described. In other words, the cap is then characterized thereby that it contains no pores larger than 50*µ*m. Thus, it will contain no macropores as hereinbefore described.

The cap, which is thus an anterior cap, may be in the form of a circular concave or dish-shaped disc integrated with or embedded in the body of bioactive material. The diameter of the rim of the cap may be the same as the diameter of the implant; however, preferably, it has a smaller diameter than that of the implant. Preferably, the diameter of the rim of the cap may be about three-quarters that of the implant.

The cap will thus be thin relative to the diameter of the implant. Thus, its thickness may be no more than half the diameter of the implant, and preferably about one-fortieth of the diameter of the implant.

While the bioactive material of the cap can, at least in principle, be different to that of the body, it is envisaged that the body and the cap will normally be of the same bioactive material. The bioactive material may, in particular, be synthetic hydroxyapatite.

The orbital implant of the invention is thus, in use, placed into an orbit of a mammal.

The placing of an orbital implant of the integrated type, ie an orbital implant which, in use, becomes integrated through tissue ingrowth and vascularization, such as that of the invention, following evisceration or enucleation, is known.

The mammal will thus be one who has had an ocular enucleation or evisceration, or who needs an implant replacement. Use of the orbital implant according to the invention will, it is believed, result in fibrovascular tissue ingrowth into the entire body of the implant, with the comparatively smooth cap resulting in little or no erosion of anterior tissue, including the conjunctiva, taking place.

After the implant has been placed into the orbit, eye muscles are typically attached to the implant, whereafter the implant is covered with tissue including conjunctiva, and a period of healing allowed during which fibrovascular tissue ingrowth into- the implant occurs. Thereafter, an artificial eye or prosthesis can be fitted over the conjunctiva, adjacent the cap of the implant. It follows thus that when the implant is placed into the orbit, it is orientated such that the cap faces anterior tissue including the conjunctiva.

The invention will now be described in more detail by way of example and with reference to the accompanying diagrammatic drawings.

In the drawings,
FIGURE 1 shows a front view of an orbital implant according to one embodiment of the invention;
FIGURE 2 shows a side view of the orbital implant of Figure 1;
FIGURE 3 shows an enlarged cross-sectional view of part of the orbital implant of Figure 1;
FIGURE 4 shows an enlarged cross-sectional view, similar to that of Figure 3, of an orbital implant according to another embodiment of the invention; and
FIGURE 5 shows a portion of the cross-sectional view of Figure 4, enlarged even further.

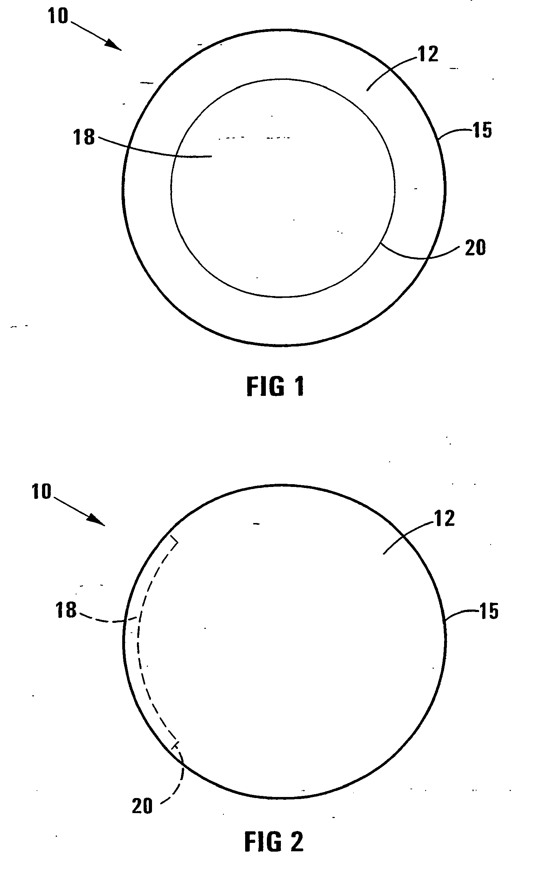

Referring to Figures 1 to 3, reference numeral 10 generally indicates an orbital implant according to one embodiment of the invention.

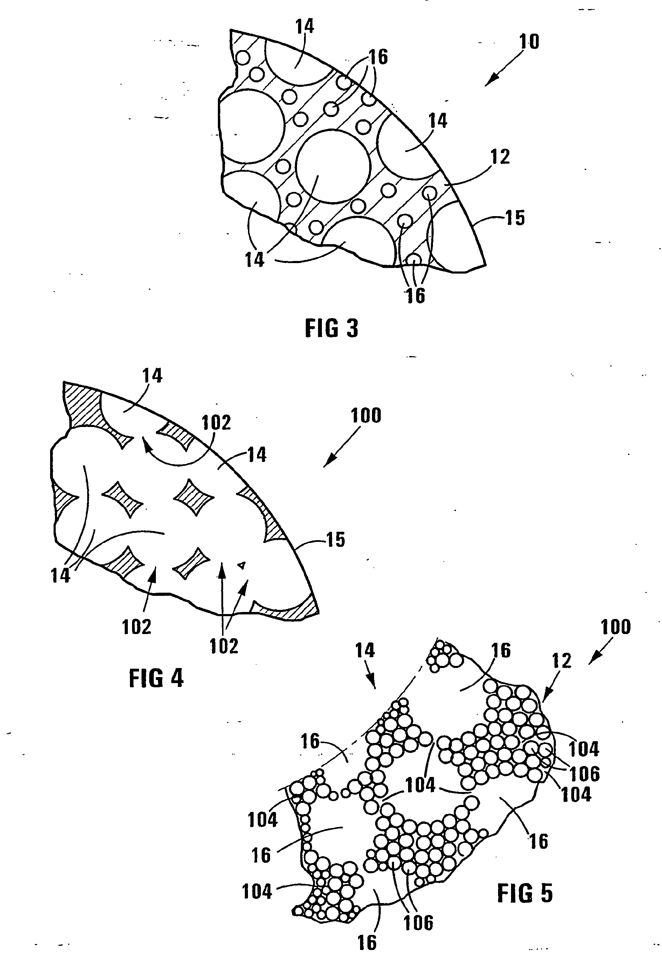

The implant 10 is substantially spherical, and has a diameter of about 20mm. It includes a body 12 of synthetic hydroxyapatite having spherical macropores 14 as well as spherical micropores 16. The macropores 14 are all of substantially the same size, and have diameters of 400-1000*µ*m, typically about 800*µ*m. The macropores 14 occupy about 60 vol % of the body 12. Some of the macropores 14 are in communication with the outer surface 15 of the body 12, as can be seen in Figure 3. It will be appreciated that at least some adjacent macropores may be interconnected (not shown) by openings or passageways (not shown).

The micropores 16 are also all of substantially the same size, and have diameters less than 50µm, eg about 10-45µm. The micropores 16 occupy about 40 vol % of the residual hydroxyapatite, ie the hydroxyapatite material between the macropores 14. The body 12 is thus solid save for the macropores and micropores therein.

The implant 10 also includes a thin anterior cap 18 of hydroxyapatite material having no macropores. The cap 1-8 thus contains either no pores at all or only micropores (not shown) having maximum dimensions less than 50*µ*m, eg having maximum dimensions of about 1*µ*m. When present, the micropores will occupy about 40% by volume of the cap material. The cap 18 is thus characterized thereby that it contains no pores larger than 50µm.

The cap 18 is in the form of a concave dish, and the rim 20 of the cap 18 has a diameter of about three-quarters that of the implant 10. Thus, when the implant 10 has a diameter of about 20mm, the rim 20 of the cap 18 has a diameter of about 15mm.

The thickness of the cap 18 is about one-fortieth the diameter of the implant 10. Thus, for an implant 10 having a 20mm diameter, the thickness of the cap 18 will be about 0.5mm.

The cap 18 thus covers only a portion of the body 12.

Referring to Figures 4 and 5, reference numeral 100 generally refers to an orbital implant according to another embodiment of the invention.

Parts of the implant 100 which are the same or similar to those of the orbital implant 10, are indicated with the same reference numerals.

The implant 100 is also substantially spherical (not shown), and has a body 12 and an anterior cap (not shown) as hereinbefore described in respect of the implant 10. The body 12 of the implant 100 also has spherical macropores 14; however, apart from some of the macropores 14 of the implant 100 being in communication with the outer surface of the body 12 of the implant 100 (as hereinbefore described in respect of the implant 10) adjacent macropores 14 are interconnected by openings 102. The diameters of the openings 102 are typically about 100*µ*m or greater. The implant 100 is normally manufactured by a sintering process such as that hereinafter described, and the interconnection of adjacent macropores then typically arises as a result of adjacent macropores coalescing together during the sintering process. As a result of the common openings 102 between adjacent macropores 14 and the macropores 14 which are in communication with the outer surface of the implant body, open paths to the body outer surface are defined by the macropores in the body 12. Thus, the body 12 contains substantially no closed or isolated macropores.

The body 12 of the implant 100 also contains spherical micropores 16 (see Figure 5), as hereinbefore described in respect of the implant 10. Moreover, it also contains irregular micropores 104 in the form of interstitial spaces between hydroxyapatite particles 106, resulting from incomplete sintering of hydroxyapatite particles 106 during formation of the body 12 by means of a sintering process such as that hereinafter described. Although the hydroxyapatite particles are shown, in Figure 5, as distinct separate particles, this is for ease of illustration only. In fact, adjacent particles will thus be partially sintered together so that such adjacent particles can no longer be viewed as being distinct particles (as shown in Figure 5) but rather merge so that they are in the form of an agglomerated mass containing the spherical macropores 14, the spherical micropores 16 and the irregular micropores 104. The sizes of the micropores 104 are substantially the same, and are dictated by the sizes of the hydroxyapatite particles 106 used for sintering. Thus, when the particle sizes are about 1*µ*m, the maximum dimensions of the micropores 104 may be about 1*µ*m, or smaller.

Adjacent micropores 16 and 104 are thus interconnected. Typically, adjacent micropores 16 are interconnected by micropores 104. Additionally, the micropores 104 and/or the micropores 16 are also interconnected to the macropores 14. Thus, the micropores 16, 104 together with the macropores 14, also define open paths to the outer surface of the implant body 12. There are thus substantially no closed or isolated micropores 16, 104 in the implant body.

The irregular micropores 104 typically occupy about 40% by volume of the residual hydroxyapatite, ie the macropore free hydroxyapatite, while the spherical micropores 16 typically occupy about 10% by volume of the residual hydroxyapatite.

To manufacture the implant 100, a mixture A is prepared by compounding hydroxyapatite powder having a mean particle size of about 1*µ*m, with a polymeric binder of a type suitable for injection moulding or extrusion; grinding the mixture to less than 300*µ*m particle size; and mixing stearic acid balls with a size distribution between 500-1000*µ*m therewith.

A mixture B is prepared by compounding hydroxyapatite powder having a mean particle size of about 1*µ*m with the same polymeric binder as used for mixture A; and grinding the mixture to less than 300*µ*m particle size. The mixture A is loaded into a die suitable for pressing of a sphere. This die includes a piston which will create a depression on the surface of the sphere during pressing, with the depression having the size and shape of the desired cap 18. The mixture A is lightly pressed to form a sphere containing the said depression. The depression is then filled with a correct amount of the mixture B. Thereafter the structure including the sphere with powder is consolidated by pressing to form a spherical compact comprising mixture A with an intimately bound cap of mixture B. The structure is sintered at a temperature below 1100°C.

It will be appreciated that when an implant in accordance with the invention is made by means of a sintering process such as that hereinbefore described, interstitial micropores 104 which result from incomplete sintering of adjacent hydroxyapatite particles, will thus normally be present. Thus, such interstitial micropores will also be present in the body 12 of the implant 10 when it is manufactured by means of such a sintering process.

The implants 10, 100 can be implanted into the orbit or eye socket of a human who has had an ocular enucleation or evisceration, or who needs an implant replacement. The implants can be placed according to known procedures for integrated implants. For example, in the case of an evisceration, the implant is implanted to replace the eye contents. Or, in the case of enucleation, the implant is placed without covering or with a covering or wrapping of tissue or artificial material into the eye muscle cone (not shown), and the eye muscles attached directly to the implant 10, 100 or to the implant wrapping. Instead, the eye muscles can be wrapped around the implant 10, 100 and secured together without direct attachment of the eye muscles to the implant 10, 100. The anterior surface of the implant is covered with tissue including the conjunctiva. The cap 18 faces the conjunctiva. A healing period is then allowed. During this healing period, fibrovascular tissue ingrowth into the entire body 12 is promoted by the bioactive hydroxyapatite surfaces in conjunction with the open paths provided by the macropores 14, the micropores 16 and the micropores 104. Following this period of healing, the implant is integrated and, due to the muscle attachment, capable of movement. Thereafter the prosthesis, ie an artificial eye, is located in position adjacent the cap 18, to obtain an artificial eye with natural appearance and good motility.

It is believed that the orbital implant of this invention addresses two common causes of complications associated with the use of orbital implants of the integrated type. These are incomplete fibrovascular tissue ingrowth into the implant interior and erosion of anterior tissue by rough surface protrusions of a porous body.

The orbital implant of this invention addresses the first of these causes of complications by promoting complete ingrowth of fibrovascular tissue into the implant interior. This is achieved by the implant of the invention having three modified material properties, as compared to properties commonly encountered in known porous orbital implants:
- Firstly, the macropore size is substantially increased, by a factor of 2 to 5, over that commonly encountered in known porous orbital implants. Macropore size is generally restricted in porous orbital implants, to achieve improved mechanical properties and an even external roundness. This is particularly important when the implant is made from materials derived from natural sources such as coral or processed bovine bone, where the external shape has to be achieved by machining. With such materials, the external roundness can be extremely uneven due to the fracture of brittle protrusions and pore edges during machining. It also produces undesirable sharp fracture surfaces. In the orbital implant of this invention, an even roundness is readily achieved due to the entirely synthetic manufacture thereof, which eliminates any need for machining of a brittle surface and thereby avoids protrusions with sharp fracture surfaces.
- Secondly, this larger macropore size is associated with a corresponding increase in the size of the interconnecting openings between adjacent macropores, to the extent that even the interconnecting openings are larger than the macropores commonly encountered in known porous orbital implants.
- Thirdly, the orbital implant of the invention can have an engineered distribution of open micropores along the macropore surfaces and in the bulk of the ceramic material. These micropores are present in a very high volume fraction, typically 40 vol % of the macropore-free hydroxyapatite material. This engineered micropore distribution distinguishes the orbital implant of this invention over known bioceramic orbital implant materials, where microporosity is either absent in the material source or regarded as detrimental to mechanical strength and therefore eliminated to a large extent. The small micropore size present at high volume fraction serves to significantly increase surface roughness at the cellular level. It further achieves an increase in surface area, up to a factor of 70, over that of an equivalent material without the micropore distribution. This is desirable in that it increases the bioactivity of the ceramic material. It further achieves a strong associated capillary force exerted by the ceramic bulk, which is absent from materials without the high level of microporosity since the force is proportional to the volume fraction of micropores and inversely proportional to the micropore size. The high degree of surface roughness, the large surface area and the strong capillary force result in immediate and strong adhesion of tissue to the material, avoiding motion and, importantly, micromotion of tissue against the implant. It further results in rapid ingress and retention of fluid with improved cell attachment. When combined with the inherent bioactive property of the hydroxyapatite composition it is further associated with direct tissue apposition, that is direct tissue ongrowth without intervening fibrous tissue as in the case of polymer materials. Finally, it is believed that the combined material properties may be associated with binding and expression of autologous growth factors at the site, which promote early tissue healing.

Thus, to summarize, the large macropore size combined with large interconnecting opening size result in open access for fluid and tissue ingrowth to the central regions of the implant. Along the inner macropore surfaces and bulk of the material, the material has been engineered to exhibit high surface roughness, high surface area, strong capillary force and inherent bioactivity. This ensures immediate strong tissue attachment, elimination of micromotion, rapid ingress and retention of fluid with improved cell attachment, direct tissue apposition without intervening fibrous tissue.

The orbital implant of this invention further addresses the second cause of complications associated with porous orbital implants of the integrated type. This is the tendency of porous materials to present a rough surface with sharp protrusions to anterior tissue, leading to erosion of the tissue and complications such as exposure of the implant. By incorporating a cap of comparative smoothness, the implant does not present sharp edges to anterior tissue. This serves to avoid erosion of the anterior tissue. The cap is an integral part of the implant structure and is comprised of the same material as the implant body, incorporating the same micropore distribution as described. Hence it exhibits a similar degree of tissue attachment, capillary force and high bioactivity as the porous ceramic body, even in the absence of macropores, since the inherent high bioactivity of the microporous hydroxyapatite allows direct tissue attachment even in the absence of significant tissue ingrowth. From a tissue engineering and materials point of view, it is significant and advantageous that a seamless transition is achieved from porous body to cap, particularly in such a sensitive location where the overlying anterior tissue is relatively thin. This fully incorporated cap serves to further distinguish the material from known orbital implants. Thus, it is different from a cap of different material over the anterior region, which will introduce an artificial transition from a tissue engineering and materials point of view, since two different materials are unlikely to evoke identical response and achieve a seamless transition. It is also different from a polymer cap, in that a polymer cap will exhibit low or no bioactivity and will require some different means to achieve attachment of the anterior tissue. It is also different from a temporary resorbable coating over the implant, such as a polymer- or inorganic cement-based cap, since a resorbable coating will merely delay ingrowth- and ongrowth to the ceramic while the underlying roughness of the porous body will eventually present again. Finally, it is extremely difficult or impossible from a ceramic processing point of view to attach and incorporate such a cap on pre-densified material, such as a coral-derived or bone-derived material.

Thus, the implant body with incorporated cap does not present sharp and rough edges to the anterior tissue, thereby avoiding erosion of the anterior tissue. Further, full incorporation of the cap is advantageous in that it presents a seamless transition from porous body to cap from a tissue engineering and materials point of view. Further, the cap material exhibits high surface area, suitable roughness at the cellular level only, strong capillary force and inherent high bioactivity. These properties jointly promote tissue attachment, elimination of micromotion, rapid ingress and retention of fluid with improved cell attachment, direct tissue apposition without intervening fibrous tissue.

## Claims

1. An orbital implant (10) which is substantially spherical, and which includes a body (12) of bioactive material having macropores (14) of at least 400µm as well as micropores (16) smaller than 50µm, with some macropores being in communication with the outer surface (15) of the body and with adjacent macropores being interconnected by openings and/or passageways so that open paths to the outer surface of the body are thereby provided, and a cap (18) of bioactive material having substantially no pores or only micropores smaller than 50µm, with the cap covering a portion of the body.

2. An implant according to Claim 1, which has a diameter of about 20mm.

3. An implant according to Claim 1 or Claim 2, wherein the macropores in the body are substantially spherical so that they have diameters of at least 400*µ*m, and, optionally, wherein the diameters of the macropores do not exceed 1000*µ*m.

4. An implant according to any one of Claims 1 to 3 inclusive, wherein substantially no isolated or closed macropores are present in the body.

5. An implant according to any one of Claims 1 to 4 inclusive, wherein the openings and/or passageways which interconnect adjacent macropores have diameters greater than 50µm.

6. An implant according to any one of Claims 1 to 5 inclusive, wherein the macropores in the body occupy from 40% to 85% by volume of the body.

7. An implant according to any one of Claims 1 to 6 inclusive, wherein at least some of the micropores are of irregular shape, and have a maximum dimension smaller than 50µm.

8. An implant according to any one of Claims 1 to 6 inclusive, wherein at least some of the micropores are substantially spherical so that their diameters are thus smaller than 50µm.

9. An implant according to any one of Claims 1 to 8 inclusive, wherein adjacent micropores in the body are interconnected by openings and/or passageways and wherein some micropores are also interconnected to the macropores by openings and/or passageways so that the micropores, by means of those openings and/or passages, provide open paths to the macropores.

10. An implant according to Claim 9, wherein substantially no isolated or closed micropores are present in the body.

11. An implant according to any one of Claims 1 to 10 inclusive, wherein some of the micropores are of irregular shape and are in the form of interstitial spaces between incompletely sintered bioactive material particles, and wherein some of the micropores are of substantially spherical shape, with irregular micropores interconnecting adjacent spherical micropores, and also connecting spherical micropores to macropores.

12. An implant according to Claim 11, wherein all the spherical micropores are of substantially the same size while all the irregular micropores are of substantially the same size, with the irregular micropores being smaller than the spherical micropores.

13. An implant according to Claim 11 or Claim 12, wherein substantially no isolated or closed micropores are present in the body.

14. An implant according to any one of Claims 1 to 13 inclusive, wherein the micropores occupy from 3% to 70% by volume of the macropore-free bioactive material.

15. An implant according to any one of Claims 1 to 14 inclusive, wherein the cap is in the form of a circular concave disc integrated with the body of bioactive material.

16. An implant according to any one of Claims 1 to 15 inclusive, wherein the cap has a thickness which is no more than half the diameter of the implant.

17. An implant according to any one of Claims 1 to 16 inclusive, wherein the bioactive material of the body and that of the cap are the same, and is hydroxyapatite.

## Patentansprüche

1. Augenhöhlenimplantat (10), das im Wesentlichen sphärisch ist und das einen Körper (12) aus bioaktivem Material umfasst, welches Makroporen (14) von mindestens 400 µm sowie Mikroporen (16) kleiner als 50 µm aufweist, wobei einige Makroporen mit der äußeren Oberfläche (15) des Körpers in Verbindung stehen und wobei benachbarte Makroporen über Öffnungen und/oder Durchgänge miteinander verbunden sind, so dass **dadurch** offene Wege zur äußeren Oberfläche des Körpers vorgesehen werden, und das eine Kappe (28) aus bioaktivem Material umfasst, welches im Wesentlichen keine Poren oder lediglich Mikroporen kleiner als 50 µm aufweist, wobei die Kappe einen Abschnitt des Körpers bedeckt.

2. Implantat nach Anspruch 1, das einen Durchmesser von ungefähr 20 mm aufweist.

3. Implantat nach Anspruch 1 oder Anspruch 2, wobei die Makroporen im Körper im Wesentlichen sphärisch sind, so dass sie Durchmesser von mindestens 400 µm aufweisen und wobei wahlweise die Durchmesser der Makroporen 1000 µm nicht überschreiten.

4. Implantat nach einem der Ansprüche 1-3, wobei im Wesentlichen keine isolierten oder geschlossenen Makroporen im Körper vorhanden sind.

5. Implantat nach einem der Ansprüche 1-4, wobei die Öffnungen und/oder Durchgänge, die benachbarte Makroporen miteinander verbinden, Durchmesser größer als 50 µm aufweisen.

6. Implantat nach einem der Ansprüche 1-5, wobei die Makroporen im Körper von 40 Volumenprozent bis 85 Volumenprozent des Körpers einnehmen.

7. Implantat nach einem der Ansprüche 1-6, wobei mindestens einige der Mikroporen eine unregelmäßige Form aufweisen und eine Maximalabmessung kleiner als 50 µm besitzen.

8. Implantat nach einem der Ansprüche 1-6, wobei mindestens einige der Mikroporen im Wesentlichen sphärisch sind, so dass ihre Durchmesser somit kleiner als 50 µm sind.

9. Implantat nach einem der Ansprüche 1-8, wobei benachbarte Mikroporen im Körper über Öffnungen und/oder Durchgänge miteinander verbunden sind und wobei einige Mikroporen auch mit den Makroporen durch Öffnungen und/oder Durchgänge verbunden sind, so dass die Mikroporen mit Hilfe jener Öffnungen und/oder Durchgänge offene Wege zu den Makroporen bereitstellen.

10. Implantat nach Anspruch 9, wobei im Wesentlichen keine isolierten oder geschlossenen Mikroporen im Körper vorhanden sind.

11. Implantat nach einem der Ansprüche 1-10, wobei einige der Mikroporen eine unregelmäßige Form besitzen und die Form von Zwischenräumen zwischen den unvollständig gesinterten Partikeln bioaktiven Materials besitzen, wobei einige der Mikroporen eine im Wesentlichen sphärische Form aufweisen, wobei unregelmäßige Mikroporen benachbarte sphärische Mikroporen miteinander verbinden und auch sphärische Mikroporen mit Makroporen verbinden.

12. Implantat nach Anspruch 11, wobei alle sphärischen Mikroporen im Wesentlichen dieselbe Größe besitzen, während alle unregelmäßigen Mikroporen im Wesentlichen dieselbe Größe besitzen, wobei die unregelmäßigen Mikroporen kleiner als die sphärischen Mikroporen sind.

13. Implantat nach Anspruch 11 oder 12, wobei im Wesentlichen keine isolierten oder geschlossenen Mirkoporen im Körper vorhanden sind.

14. Implantat nach einem der Ansprüche 1-13, wobei die Mikroporen von 3 Volumenprozent bis 70 Volumenprozent des Makroporen freien bioaktiven Materials einnehmen.

15. Implantat nach einem der Ansprüche 1-14, wobei die Kappe die Form einer kreisförmigen konkaven Scheibe besitzt, die mit dem Körper des bioaktivem Materials integriert ist.

16. Implantat nach einem der Ansprüche 1-15, wobei die Kappe eine Dicke besitzt, die nicht mehr als die Hälfte des Durchmessers des Implantats beträgt.

17. Implantat nach einem der Ansprüche 1-16, wobei das bioaktive Material des Körpers und jenes der Kappe dasselbe sind und dies Hydroxyapatit ist.

## Revendications

1. Implant orbitaire (10) qui est sensiblement sphérique, et qui inclut un corps (12) en matériau bioactif comportant des macropores (14) ayant une taille d'au moins 400 µm, de même que des micropores (16) ayant une taille inférieure à 50 µm, certains macropores étant en communication avec la surface extérieure (15) du corps et avec des macropores adjacents qui sont liés mutuellement par des ouvertures et/ou des passages de façon à ainsi établir des trajets ouverts vers la surface extérieure du corps, et un élément de recouvrement (18) en matériau bioactif ne comportant sensiblement pas de pores ou uniquement des micropores de taille inférieure à 50 µm, l'élément de recouvrement couvrant une partie du corps.

2. Implant selon la revendication 1, qui a un diamètre d'environ 20 mm.

3. Implant selon la revendication 1 ou la revendication 2, dans lequel les macropores réalisés dans le corps sont sensiblement sphériques de sorte qu'ils ont des diamètres d'au moins 400 µm et, de façon facultative, dans lequel les diamètres des macropores ne dépassent pas 1 000 *µ*m.

4. Implant selon l'une quelconque des revendications 1 à 3 incluse, dans lequel le corps ne possède sensiblement pas de macropores isolés ou fermés.

5. Implant selon l'une quelconque des revendications 1 à 4 incluse, dans lequel les ouvertures et/ou les passages qui mettent en communication mutuelle des macropores adjacents ont des diamètres supérieurs à 50 µm.

6. Implant selon l'une quelconque des revendications 1 à 5 incluse, dans lequel les macropores réalisés dans le corps occupent de 40 % à 85 % par volume du corps.

7. Implant selon l'une quelconque des revendications 1 à 6 incluse, dans lequel au moins certains des micropores ont une forme irrégulière, et ont une dimension maximale inférieure à 50 µm.

8. Implant selon l'une quelconque des revendications 1 à 6 incluse, dans lequel au moins certains des micropores sont sensiblement sphériques de sorte que leurs diamètres sont ainsi inférieurs à 50 µm.

9. Implant selon l'une quelconque des revendications 1 à 8 incluse, dans lequel les micropores adjacents réalisés dans le corps sont liés mutuellement par des ouvertures et/ou par des passages, et dans lequel certains micropores sont également en communication mutuelle avec les macropores par des ouvertures et/ou des passages de sorte que les micropores, au moyen de ces ouvertures et/ou passages, établissent des trajets ouverts avec les macropores.

10. Implant selon la revendication 9, dans lequel le corps ne possède sensiblement pas de micropores isolés ou fermés.

11. Implant selon l'une quelconque des revendications 1 à 10 incluse, dans lequel certains des micropores ont une forme irrégulière et ont la forme d'espaces interstitiels entre des particules de matériau bioactif incomplètement frittées, et dans lequel certains des micropores ont une forme sensiblement sphérique, des micropores irréguliers mettant en communication mutuelle des micropores sphériques adjacents, et mettant également en communication des micropores sphériques et des macropores.

12. Implant selon la revendication 11, dans lequel tous les micropores sphériques ont sensiblement la même taille, et tous les micropores irréguliers ont sensiblement la même taille, les micropores irréguliers étant plus petits que les micropores sphériques.

13. Implant selon la revendication 11 ou la revendication 12, dans lequel le corps ne possède sensiblement pas de micropores isolés ou fermés.

14. Implant selon l'une quelconque des revendications 1 à 13 incluse, dans lequel les micropores occupent de 3 % à 70 % par volume du matériau bioactif dépourvu de macropores.

15. Implant selon l'une quelconque des revendications 1 à 14 incluse, dans lequel l'élément de recouvrement a la forme d'un disque concave circulaire intégré au corps de matériau bioactif.

16. Implant selon l'une quelconque des revendications 1 à 15 incluse, dans lequel l'élément de recouvrement a une épaisseur qui n'est pas supérieure à la moitié du diamètre de l'implant.

17. Implant selon l'une quelconque des revendications 1 à 16 incluse, dans lequel le matériau bioactif du corps et celui de l'élément de recouvrement sont les mêmes, et est constitué d'hydroxyapatite.
